# EUROPEAN PATENT APPLICATION

(11) **EP 1 702 627 A1**
(43) Date of publication of application: **20.09.2006**
(21) Application number: 05380050.4
(22) Date of filing: 18.03.2005
(51) Int. Cl.: A61K 45/06, A61K 31/529, A61K 31/485, A61P 25/04, A61P 25/18, A61P 25/22, A61P 25/30

(54) **Analgesic combination of sodium channel blockers with opioid antagonists**

(71) Applicant: LABORATORIOS DEL DR. ESTEVE, S.A., 08041 Barcelona (ES)
(72) Inventor: Buschmann, Helmut Dr. Laboratorios Dr. Esteve S.A., 08041 Barcelona (ES)
(74) Representative: Bernardo Noriega, Francisco

(57) **Abstract**

The present invention refers to a new combination of sodium channel blockers, especially Tetrodotoxin, and their derivatives with opioid antagonists, especially naloxone, according medicinal products for human and/or animal therapeutics and their use for the treatment of a variety of diseases, especially pain, preferably neuropathic pain.

## Description

### Field of the invention

The present invention refers to a new combination of sodium channel blockers, especially Tetrodotoxin, and their derivatives with opioid antagonists, especially naloxone, according medicinal products for human and/or animal therapeutics and their use for the treatment of a variety of diseases, especially pain, preferably neuropathic pain.

### Background of the invention

The treatment of pain conditions is of great importance in medicine. There is currently a world-wide need for additional pain therapy. The pressing requirement for a specific treatment of pain conditions or as well a treatment of specific pain conditions which is right for the patient, which is to be understood as the successful and satisfactory treatment of pain for the patients, is documented in the large number of scientific works which have recently and over the years appeared in the field of applied analgesics or on basic research on nociception.

PAIN is defined by the International Association for the Study of Pain (IASP) as "an unpleasant sensory and emotional experience associated with actual or potential tissue damage, or described in terms of such damage (IASP, Classification of chronic pain, 2nd Edition, IASP Press (2002), 210). Even though pain is always subjective its causes or syndromes can be classified.

Especially neuropathic pain, i.e. pain caused by lesions of the peripheral or central nervous system, is still a challenge for clinicians as no specific drugs actually exist for its alleviation. Indeed, the use of antidepressants and anticonvulsants to reduce neuropathic pain is founded on empirical observations only, and the efficacy of these drugs is most often limited both in amplitude and duration. Furthermore, patients often complain of undesirable side effects and develop tolerance to the anti-pain action of these drugs. Therefore, novel therapeutic strategies have to be developed, possibly on a rationale basis.

Therefore, it was the underlying problem of this invention to find new ways of treating pain, especially neuropathic pain.

So, the main object of this invention is a combination of pharmaceutically active compounds comprising:
at least one Compound A) selected from a Sodium channel blocker, and/or of one of its derivatives, optionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate;
   and
at least one Compound B) selected from an opioid antagonist optionally in the form of its racemate, and/or one of its derivativesoptionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate.

It was found out that the combination of Sodium channel blocker and opioid antagonists are acting surprisingly strong on pain, especially neuropathic pain with a completely unforeseeable over additive effect. In addition there seems to be an impressive effect of the combination on addiction, especially opiod addiction.

The term "sodium channel blocker" mentioned in this application is defined as a compound that specifically binds to and specifically inhibits sodium channels, especially TTX-resistant or TTX-sensitive sodium channels. The term TTX-resistant" and TTX-sensitive refers to a difference in the tightness of TTX binding, with the TTX resistant channel having a binding constant as mentioned in Hunter et al.,Current Opinion in CPNS Investigational Drugs 1 (1), 1999 as well as Clare et al. DDT, 5 (11), 2000, 506-520 included here by reference and the TTX sensitive channel having a binding as mentioned in constant Hunter et al., Current Opinion in CPNS Investigational Drugs 1 (1), 1999 as well as Clare et al. DDT, 5 (11), 2000, 506-520. A preferred sodium channel blocker thus binds to a sodium channel with a Ki of less than 200 µM, preferably less than 100 µM or with an IC₅₀ of 2 µM. Said inhibition refers to suppression or modification of any downstream effect caused by activation of said sodium channels. More preferably, the term "sodium channel blocker" mentioned in this invention refers to compounds binding to an alpha subunit of sodium channels, especially TTX-resistant or TTX-sensitive sodium channels. More preferably, the term "sodium channel blocker' mentioned in this invention refers to compounds binding to either a SS1 or SS2 region of an alpha subunit of sodium channels, especially TTX-resistant or TTX-sensitive sodium channels. Preferred sodium channel blockers for use in this invention are tetrodotoxin and saxitoxin which both specifically inhibit said sodium channels.

Opioids is the common name for all compounds which have the same mode of action as the constituents of opium, the dried milky liquid of the poppy seed, Papaver somniferum (Brownstein, 1993). All opioids interact in biological systems with the same type of receptor, the so-called opioid receptor. The opioid peptide systems plays an important role in pain and is significantly implicated in antinociceptive processes. The main groups of opioid peptides are enkephalins, dynorphins and beta-endorphin which derive from proenkephalin, prodynorphin and proopiomelanocortin, respectively. Three types of opioid receptors have been cloned - µ, δ, and κ (Evans et al (1992) Science 258 1952; Kieffer et al (1992) Proc.Natl.Acad.Sci.USA 89 12048; Chen et al (1993) Mol.Pharmacol. 44 8, Minami et al (1993) FEBS Lett. 329 291). More recently, a fourth opioid receptor type, named ORL1-receptor has been added (Meunier et al., 1995). Within the µ- and δ-receptor type 2 subtypes, the µ-1 and µ-2 and δ-1 and δ-2 have been described. The ?-receptor contains an additional ?-3 subtype. All opioid receptors belong to the group of pertussis toxin-sensitive G protein coupled receptors of the rhodopsine family with seven transmembrane spanning hydrophobic domains. The peptide structure of the four different opioid receptors is very similar (Gaveriaux-Ruff and Kieffer, 1999) and this is the reason why most of the opioid receptor ligands show affinity for more than one type of receptor. In most cases, one type is preferred and the action and side-effect profile is dominated by the properties of this receptor. Intensive research was necessary to find out receptor-specific 'selective' ligands, but several selective agonists and antagonists for each type of opioid receptor have now been developed.

In regard to this invention the term "opioid antagonist" refers to a molecule or a compound which has affinity to an opioid receptor and inhibits, modifies or reduces the intensity of its downstream signalling. Additionally, the term "opioid antagonist" refers to a compound which shows at least 50 % inhibition at a concentration of 10µM in radioactive replacement experiments or has at least a Ki value of 1 µM towards an opioid receptor. Opioid receptors referred to in this invention are µ, κ, δ or ORL-1 receptor. Examples of opioid antagonists include but are not limited to Naloxone, Naltrexone, Etonitazenyl isothiocyanate, Naloxonazine dihydrochloride, Clocinnamox mesylate, b-Funaltrexamine hydrochloride, CTOP or CTAP.

The term "analogues" as used in this application is defined here as meaning a chemical compound structurally related to an acting compound having similar activity.

The term "derivatives" as used in this application is defined here as meaning a chemical compound having undergone a chemical derivation starting from a acting compound e.g. a µ-opioid antagonist, to change (ameliorate for pharmaceutical use) any of its physico-chemical properties, especially a so-called prodrug, e.g. their esters and ethers. Examples of well known methods of producing a prodrug of a given acting compound are known to those skilled in the art and can be found e.g. in Krogsgaard-Larsen et al., Textbook of Drugdesign and Discovery, Taylor & Francis (April 2002).

In connection with this invention "neutral form" refers to the non-ionic form but also to (at its isoelectric point) neutrally charged forms (that means containing an equal amount of positive and negative charges) especially the Zwitter-lon.

The term "salt" according to this invention is to be understood as meaning any form of the active compound according to the invention in which this compound assumes an ionic form or is charged and - if applicable - is also coupled with a counter-ion (a cation or anion). By this are also to be understood complexes of the active compound with other molecules and ions, in particular complexes which are complexed via ionic interactions. As preferred examples of salts this includes the acetate, mono-trifluoracetate, acetate ester salt, citrate, formate, picrate, hydrobromide, monohydrobromide, monohydrochloride or hydrochloride.

The term "physiologically acceptable salt" in the context of this invention is understood as meaning a "salt" (as defined above) of at least one of the compounds according to the invention which are physiologically tolerated - especially if used in humans and/or mammals.

The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

Another preferred aspect of the invention is a combination of pharmaceutically active compounds consisting of:
at least one Compound A) selected from a Sodium channel blocker, and/or of one of its derivatives, optionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate;
   and
at least one Compound B) selected from an opioid antagonist optionally in the form of its racemate, and/or one of its derivativesoptionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate physiologically acceptable salt, or in form of a solvate, especially a hydrate.

Another preferred aspect of the invention is a combination according to the invention in that at least one compound A is a sodium channel blocker specific for a TTX-sensitive Sodiumchannel.

The term "specific" means, in the case of TTX, binding to one subtype (TTX-sensitive or TTX-resistant) of a sodium channel with an IC50 lower than for the other subtype and, in the case of an opioid antagonist, binding with a lower Ki value for any of opioid receptor subtypes (µ, κ, δ or ORL-1) than for the other opioid receptor subtypes.

The term "TTX-sensitive sodium channel" refers to a type of voltage-gated sodium channel with IC₅₀ values of TTX being in the range of 1 to 12 nM, as published in the IUPHAR Compendium of Voltage-gated lon Channels (2002). TTX-sensitive sodium channels include the Naᵥ1.1, Naᵥ1.2, Naᵥ1.3, Naᵥ1.6, and Naᵥ1.7.

Another preferred aspect of the invention is a combination according to the invention in that at least one compound A is a sodium channel blocker specific for a TTX-resistant Sodiumchannel.

The term "TTX-resistant sodium channel" refers to a type of voltage-gated sodium channel with IC₅₀ values of TTX being above 1 µM, as published in the IUPHAR Compendium of Voltage-gated Ion Channels (2002). TTX-resistant sodium channels include the Naᵥ1.4, Naᵥ1.5, Naᵥ1.8 and Naᵥ1.9.

Another preferred aspect of the invention is a combination according to the invention in that at least one compound A is selected from Tetrodotoxin and/or at least one of its analogues or derivatives optionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate;
and/or from Saxitoxin and/or at least one of its analogues or derivatives optionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate.

Tetrodotoxin (alternatively in the context of this application abbreviated TTX), also known as Ti Qu Duo Xin, is an alkaloid found in puffer fish (Tetradontiae). The chemical name is Octahydro-12-(Hydroxymethyl)-2-imino-5, 9, 7, 10a-dimethano-10aH-[1,3]dioxocino[6,5-d]pyrimidine-4,7,10,11,12-pentol with a molecular formula C₁₁H₁₇N₃O₈ and a Molecular weight of 319.27. It is a potent non-protein neurotoxin and an indispensable tool for the study of neurobiology and physiology.

Tetrodotoxin (TTX) is a marine organic toxin which is mainly found in testicles, ovaries, eggs, livers, spleens, eyeballs, and blood of puffer fish as well as in diverse animal species, including goby fish, newt, frogs and the blue ringed octopus and even in marine alga. Several processes for producing TTX are known. Usually TTX is extracted from marine organisms (e.g. JP 270719 Goto and Takahashi) but besides numerous others methods of synthesis are also described (and used for the preparation of tetrodotoxin in connection to this invention) in US 6,552,191, US6,478,966, US 6,562,968 or 2002/0086997, all of which are included here by reference. Tetrodotoxin is a well known compound described for example in WO02/22129 as systemically acting as analgesic. For one of the many descriptions of TTX it is recommended turn to e.g. Tu, Anthony (Ed.) Handbook of Natural Toxins, Vol. 3: Marine Toxins and Venoms, 1988, 185-210 as well as Kao (1966), Pharmacol. Rev. 18:997 - 1049 and others.

Older journals describe that based on the method described by Tahara in US 1,058,643, there was a product sold in Japan containing a 1% solution of TTX extract for uses such as enuresis and others (Iwakawa and Kimura, Archiv fuer Experimentelle Pathologie und Pharmakologie (1922), 93, 305-31). In parallel there were trials in the 1930s (Hsiang, Nai Shi; Manshu Igaku Zasshi (1939), 30, 639-47 (German abstr. 179) testing the abilities of TTX for the treatment of addiction to opioids.

Tetrodotoxin is a well known compound described for example in CN 1145225 as acting as an analgesic as well as in the treatment of drug addiction. WO02/22129 describes TTX as systemically acting as an analgesic, including acting on neuropathic pain. This general mentioning of neuropathic pain as an example of pain to be treated with TTX is not going into any details regarding neuropathic pain especially no mentioning of any subtype.

The phrase "its (tetrodoxin's) derivatives and analogues" according to this invention is defined - using the definition of US 6,030,974 (included here by reference) - as meaning amino perhydroquinazoline compounds having the molecular formula C₁₁H₁₇N₃O₈. "Tetrodoxin's derivatives and analogues" according to this invention encompasses compounds described in US 5,846,975 (included here by reference) as amino hydrogenated quinazolines and derivatives including the substances set forth from column 3 line 40 to column 6 line 40. Specifically exemplified "derivatives and analogues of tetrodotoxin" according to this invention are including but are not limited to anhydro-tetrodotoxin, tetrodaminotoxin, methoxytetrodotoxin, ethoxytetrodotoxin, deoxytetrodotoxin and tetrodonic acid, 6 epi-tetrodotoxin, 11-deoxytetrodotoxin as well as the hemilactal type TTX analogues (e.g. 4-epi-TTX, 6-*epi*-TTX, 11-*deoxy*-TTX, 4-*epi*-11-*deoxy*-TTX, TTX-8-*O*-hemisuccinate, chiriquitoxin, 11-*nor*-TTX-6(S)-ol, 11-*nor-*TTX-6(R)-ol, 11-*nor*-TTX-6,6-diol, 11-*oxo-*TTX and TTX-11-carboxylic acid), the lactone type TTX analogues (e.g. 6-*epi*-TTX (lactone), 11-*deoxy*-TTX (lactone), 11-*nor*-TTX-6(S)-ol (lactone), 11-*nor*-TTX-6(R)-ol (lactone), 11-*nor*-TTX-6,6-diol (lactone), 5-*deoxy*-TTX, 5,11-*dideoxy*-TTX, *4-epi-*5,11-*didroxy*-TTX, 1*-hydroxy-*5,11*-dideoxy*-TTX, 5,6,11-*trideoxy*-TTX and 4-epi-5,6,11-*trideoxy*-TTX) and the 4,9-anhydro type TTX analogs (e.g. 4,9-*anhydro*-TTX, 4,9*-anhydro-6-epi-*TTX*,* 4,9*-anhydro-*11*-deoxy-*TTX*,* 4,9-*anhydro*-TTX-8-O-hemisuccinate, 4,9-*anhydro*-TTX-11-O-hemisuccinate). The typical analogues of TTX possess only 1/8 to 1/40 of the toxicity of TTX in mice, based upon bioassay in mice. It has been observed that the analogues produce joint action, and do not interact adversely. Examples of TTX analogues include novel TTX analogs isolated from various organisms, as well as those that are partially or totally chemically synthesized (see e.g., Yotsu, M. et al. Agric. Biol. Chem., 53(3):893-895 (1989)). "Analogues" of TTX bind to the same site on the alpha subunit of sodium channels as does TTX.

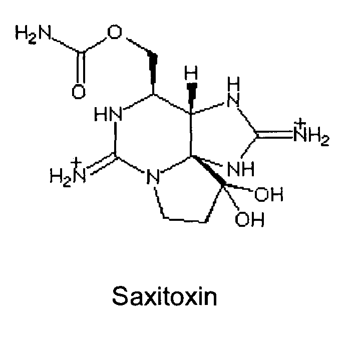

Saxitoxin is - according to the Merck Index on CD Version 12:1 - a mussel poison; clam poison; paralytic shellfish poison; gonyaulax toxin. This powerful neurotoxin is produced by the dinoflagellates Gonyaulax catenella, or G. tamarensis, the consumption of which causes the California sea mussel Mytilus californianus, the Alaskan butterclam Saxidomus giganteus and the scallop to become poisonous: Sommer et al., Arch. Pathol. 24, 537, 560 (1937); Schantz et al., Can. J. Chem. 39, 2117 (1961); Ghazarossian et al., Biochem. Biophys. Res. Commun. 59, 1219 (1974). These poisonous shellfish have been connected to instances of toxic "red-tides" where the high concentrations of algae discoloring the water were of the Gonyaulax genus. Isoln and partial characterization: Schantz et al., J. Am. Chem. Soc. 79, 5230 (1957); Mold et al., ibid. 5235. Saxitoxin is a very popular tool in neurochemical research and as a sodium channel blocker is recently being described in a number of therapeutic uses.

In a preferred embodiment of the invention the combination according to the invention is defined in that Compound A is selected from tetrodotoxin, optionally in the form of its racemate, pure stereoisomers, especially enantiomers or diastereomers or in the form of mixtures of stereoisomers, especially enantiomers or diastereomers, in any suitable ratio;in neutral form, in the form of an acid or base or in form of a salt, especially a physiologically acceptable salt, or in form of a solvate, especially a hydrate.

In a very preferred embodiment of the invention the combination according to the invention is defined in that Compound A is selected from tetrodotoxin in neutral form or as a salt, especially a physiologically acceptable salt.

In a preferred embodiment of the invention the combination according to the invention is defined in that in that Compound A is selected from tetrodotoxin, its derivative or its analogue which is isolated from a biological source, preferably from fish, especially puffer fish.

In a preferred embodiment of the invention the combination according to the invention is defined in that Compound A is selected from tetrodotoxin, its derivative or its analogue which is synthesized.

In a preferred embodiment of the invention the combination according to the invention is defined in that at least one Compound B is an unspecific opioid antagonist, a specific µ-opioid antagonist, a specific ORL1-antagonist, a specific δ-opioid antagonist, or a specific κ-opioid antagonist, including their derivatives optionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate.

In a preferred embodiment of the invention the combination according to the invention is defined in that Compound in that at least one Compound B is a specific δ-opioid antagonist, including the derivatives optionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate.

In a preferred embodiment of the invention the combination according to the invention is defined in that at least one Compound B is a specific µ-opioid antagonist, including their derivatives optionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate.

In a preferred embodiment of the invention the combination according to the invention is defined in that at least one Compound B is naloxone, Naltrexone, Etonitazenyl isothiocyanate, Naloxonazine dihydrochloride, Clocinnamox mesylate, b-Funaltrexamine hydrochloride, CTOP or CTAP.

In a highly preferred embodiment of the invention the combination according to the invention is defined in that Compound A is Tetrodotoxin and Compound B is a µ-opioid antagonist.

In a highly preferred embodiment of the invention the combination according to the invention is defined in that Compound A is Tetrodotoxin and Compound B is a δ-opioid antagonist.

In a highly preferred embodiment of the invention the combination according to the invention is defined in that Compound A is Tetrodotoxin and Compound B is Naloxone, Naltrexone, Etonitazenyl isothiocyanate, Naloxonazine dihydrochloride, Clocinnamox mesylate, b-Funaltrexamine hydrochloride, CTOP or CTAP.

Another aspect of this invention is a Pharmaceutical formulation containing a combination according to the invention and optionally at least one auxiliary material and/or additive.

In a preferred embodiment of this Pharmaceutical formulation according to the invention the Compound A, especially tetrodotoxin, is present in the amount of between 10 µg and 4 mg, especially between 5 and 2000 µg, preferably between 250 and 1000 µg or between 25 and 50 µg.

In a preferred embodiment of this Pharmaceutical formulation according to the invention the Compound B, especially naloxone, is present in the amount of between 10 and 4000 mg/day, preferably between 20 to 2000 mg/day, most preferably between 40 and 500 mg/day.

In another preferred embodiment of the Pharmaceutical formulation according to the invention the ratio between Compound A, especially tetrodotoxin, and Compound B, especially naloxone, in the Pharmaceutical formulation is between 1 : 2000 up to 1 : 10, preferably between 1: 500 up to 1 : 50, most preferably between 1: 300 up to 1 : 80.

In human therapeutics, the dose administered is normally between 10 and 4000 µg/day of the sodium channel blocker, especially tetrodotoxin, its derivatives or its analogues, especially the dose of tetrodotoxin administered is normally between 10 and 4000 µg/day or - given the likely twice per day treatment - between 5 to 2000 µg each given dose, sometimes preferably between 250 and 1000 µg each given dose, sometimes preferably between 25 and 50 µg each given dose depending on the route of administration.

In human therapeutics, the dose administered of the opiod antagonist is normally between 10 and 4000 mg/day, preferably between 20 to 2000 mg/day, most preferably between 40 and 500 mg/day or - given the likely twice per day treatment - between 5 to 2000 mg each given dose, sometimes preferably between 10 and 1000 mg each given dose, sometimes preferably between 20 and 250 mg each given dose depending on the route of administration. Especially for Naloxone the proper dose is well known in the art.

In connection with this invention any amount defined refers to each compound individually not to any combination and refers to the compound having a purity of ≥ 97%. This on the other hand will exclude any impurity contained within the >3% to be mentioned, defined or referred to as active compound in the sense of this invention. For example this would mean that a formulation containing 0.5 mg tetrodotoxin of 99 % purity and 0.8 % anhydro-tetrodotoxin will be classified and defined according to this invention as containing just tetrodotoxin as active ingredient.

The auxiliary material and/or additive can be specifically selected from conserving agents, emulsifiers and/or carriers for parenteral application. The selection of these auxiliary materials and/or additives and of the amounts to be used depends upon how the pharmaceutical composition is to be applied. Examples include here especially parenteral like intravenous subcutaneous or intramuscular application formulations but which could also be used for other administration routes.

Routes of administration of tetrodotoxin its derivatives and its analogues can include intramuscular injection, intraveneous injection, subcutaneous injection, sublingual, bucal, patch through skin, oral ingestion, implantable osmotic pump, collagen implants, aerosols or suppository.

Another aspect of this invention refers to a process for the production of a combination according to the invention in which Compounds A and B are mixed.

Another aspect of this invention refers to a process for the production of a pharmaceutical formulation according to the invention in which the Compounds A and B are combined during the process of formulating the pharmaceutical formulation.

Another aspect of this invention refers to the use of a combination according to the invention for the production of a medicament for the treatment of pain, especially moderate to severe pain, cancer pain (resulting from cancer), and neuropathic pain.

In a preferred embodiment this use for pain according to the invention is for neuropathic pain.

"Neuropathic pain" is defined by the IASP as "pain initiated or caused by a primary lesion or dysfunction in the nervous system" (IASP, Classification of chronic pain, 2nd Edition, IASP Press (2002), 210). For the purpose of this invention included under this heading or to be treated as synonymous is "Neurogenic Pain" which is defined by the IASP as "pain initiated or caused by a primary lesion, dysfunction or transitory perturbation in the peripheral or central nervous system".

In a preferred embodiment of the use according to the invention the neuropathic pain is central pain.

According to the IASP "central pain" is defined as "a pain initiated or caused by a primary lesion or dysfunction in the central nervous system" (IASP, Classification of chronic pain, 2nd Edition, IASP Press (2002), 211).

In another embodiment of the use according to the invention the neuropathic pain is peripheral neuropathic pain or peripheral neurogenic pain.

According to the IASP "Peripheral neuropathic pain" is defined by the IASP as "pain initiated or caused by a primary lesion or dysfunction in the peripheral nervous system" and "Peripheral neurogenic pain" as "pain initiated or caused by a primary lesion, dysfunction or transitory perturbation in the peripheral nervous system" (IASP, Classification of chronic pain, 2nd Edition, IASP Press (2002), 213).

In another preferred embodiment of the use according to the invention the neuropathic pain is allodynia.

According to the IASP "allodynia" is defined as "a pain due to a stimulus which does not normally provoke pain" (IASP, Classification of chronic pain, 2nd Edition, IASP Press (2002), 210).

In another preferred embodiment of the use according to the invention the neuropathic pain is causalgia.

According to the IASP "causalgia" is defined as "a syndrome of sustained burning pain, allodynia and hyperpathia after a traumatic nerve lesion, often combined with vasomotor and sudomotor dysfunction and later trophic changes" (IASP, Classification of chronic pain, 2nd Edition, IASP Press (2002), 210).

In another preferred embodiment of the use according to the invention the neuropathic pain is hyperalgesia.

According to the IASP "hyperalgesia" is defined as "an increased response to a stimulus which is normally painful(IASP, Classification of chronic pain, 2nd Edition, IASP Press (2002), 211).

In another preferred embodiment of the use according to the invention the neuropathic pain is hyperesthesia.

According to the IASP "hyperesthesia" is defined as "increased sensitivity to stimulation, excluding the senses" (IASP, Classification of chronic pain, 2nd Edition, IASP Press (2002), 211).

In another preferred embodiment of the use according to the invention the neuropathic pain is hyperpathia.

According to the IASP "hyperpathia" is defined as "a painful syndrome characterized by an abnormally painful reaction to a stimulus, especially a repetitive stimulus, as well as an increased threshold" (IASP, Classification of chronic pain, 2nd Edition, IASP Press (2002), 212).

The IASP draws the following difference between "allodynia", "hyperalgesia" and "hyperpathia" (IASP, Classification of chronic pain, 2nd Edition, IASP Press (2002), 212):

| | | |
|---|---|---|
| Allodynia | Lowered threshold | Stimulus and response mode differ |
| Hyperalgesia | Increased response | Stimulus and response rate are the same |
| Hyperpathia | Raised threshold; Increased response | Stimulus and response rate may be the same or different |

In another preferred embodiment of the use according to the invention the neuropathic pain is neuralgia.

According to the IASP "neuralgia" is defined as "Pain in the distribution of a nerve or nerves" (IASP, Classification of chronic pain, 2nd Edition, IASP Press (2002), 212).

In another preferred embodiment of the use according to the invention the neuropathic pain is neuritis.

According to the IASP "neuritis" is defined as "Inflammation of a nerve or nerves" (IASP, Classification of chronic pain, 2nd Edition, IASP Press (2002), 212).

In another preferred embodiment of the use according to the invention the neuropathic pain is neuropathy.

According to the IASP "neuritis" is defined as "a disturbance of function or pathological change in a nerve: in one nerve mononeuropathy, in several nerves mononeuropthy multiplex, if diffuse and bilateral, polyneuropathy" (IASP, Classification of chronic pain, 2nd Edition, IASP Press (2002), 212).

In another preferred embodiment of the invention is the use of the combination according to the invention for the production of a medicament for the prophylaxis and/or treatment of drug abuse and/or drug addiction, medicament abuse and/or medicament addiction, preferably drug abuse and/or drug addiction.

Medicaments/drugs, which are frequently the subject of misuse (leading often to addiction) include opioids, especially morphine, barbiturates, cannabis, cocaine, amphetamines, phencyclidine, hallucinogens and benzodiazepines.

The term "treatment" or "to treat" in the context of this specification means administration of a compound or formulation according to the invention to prevent, ameliorate or eliminate one or more symptoms associated with de-addiction from the drug/medicament. Such symptoms can arise from withdrawal, or can be associated with relapse behavior, such as a craving of a subject for the drug/medicament. "Treatment" also encompasses preventing, ameliorating or eliminating the physiological sequelae of removal of the drug/medicament from an addicted subject that may cause the exhibited symptoms perceived by the subject. "Treatment" also encompasses a reduction in the amount of the drug/medicament that is consumed by a subject.

The term "effects of a de-addiction treatment" in the context of this invention is understood as including any side effect coming with any de-addiction treatment, especially any kind of withdrawal syndrome.

The term "ameliorate" in the context of this invention is understood as meaning any improvement on the situation of the patient treated - either subjectively (feeling of or on the patient) or objectively (measured parameters).

In another preferred embodiment of the invention is the use of the combination according to the invention for the production of a medicament for the treatment of anxiety and schizophrenia or schrizophrenic diseases.

In another preferred embodiment of the use according to the invention the Compound A is used at 10 and 4000 µg/day, preferably between 5 to 2000 µg/day.

In another preferred embodiment of the use according to the invention the Compound B is used at 10 and 4000 mg/day, preferably between 20 to 2000 mg/day.

In another preferred embodiment of the combination according to the invention the ratio between Compound A and Compound B is between 1 : 2000 up to 1 : 10, preferably between 1: 500 up to 1 : 50, most preferably between 1: 300 up to 1 : 80.

Included in this invention are especially also methods of treatments of a patient or a mammal, including men, suffering from pain or from the consequences of drug addiction, especially from neuropathic pain, using using/administering to the patient a combination or pharmaceutical formulation according to the invention. In this context of methods of treatment the term "combination" includes but does not exclusively mean the concomitant administration of compounds, but also the sequential administration of each of the compounds, as well as administration of one compound, followed by administration of the other compound, whereas the time interval between the two administrations can be between 1 min and 1 week. Additionally and most preferably though, in this context of methods of treatment "combination" refers to a formulation containing all compounds in its respective carrier such as a tablet, capsule, a pill or a gum. Most preferably, in this context of methods of treatment "combination" means a fixed combination of compounds in a single dosage form such as a tablet, a pill, a capsule or a gum. It is also preferred if the method of treatment is restricted to tetrodotoxin as Compound A and a µ-opioid receptor antagonist as Compound B, whereas tetrodotoxin, its derivative and/or one of its analogues is used in an amount between 10 µg/day and 4 mg/day and is isolated from a biological source, preferably from fish, especially puffer fish, or is synthesized and the compound B is used in an amount between 10 mg/day and 4000 mg/day.

Another preferred embodiment of the invention is a kit comprising a drug comprising a substance A selected from the group of sodium channel blockers, and/or one of its derivatives, optionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate,
and a drug comprising substance B, selected from an opioid antagonist optionally in the form of its racemate, and/or one of its derivativesoptionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate.

The examples and figures in the following section describing pharmacological trials are merely illustrative and the invention cannot be considered in any way as being restricted to these applications.

### Figures:

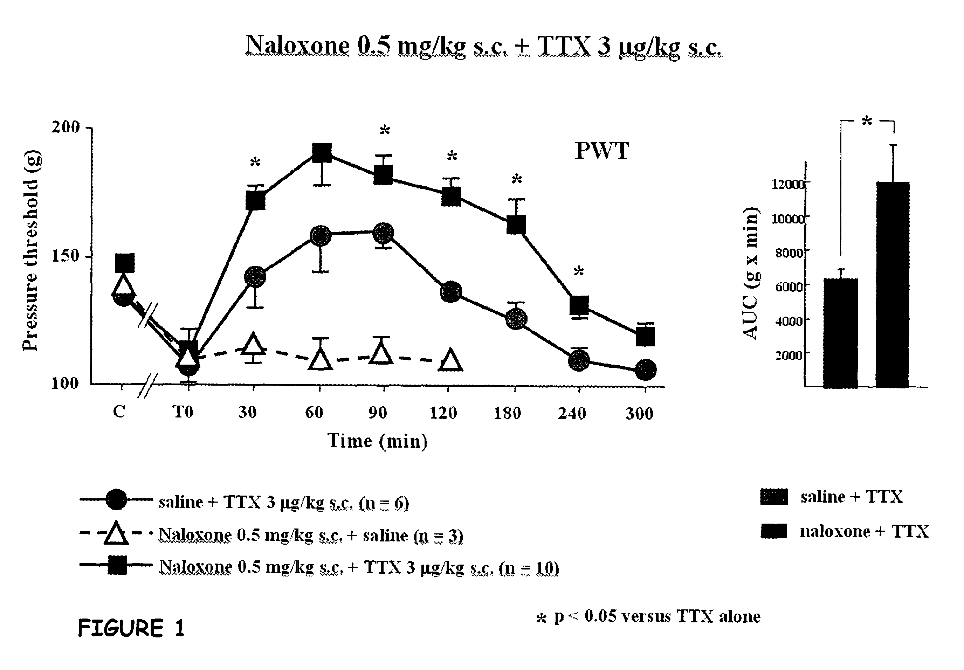

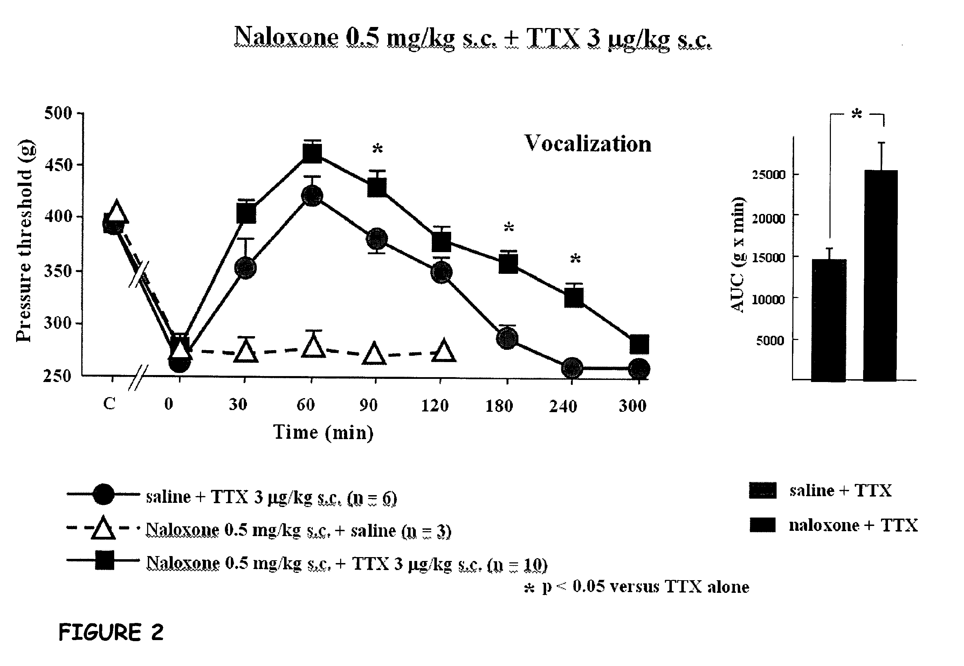

Figures 1 and 2 refer to examples 1 and 2 and are described below.

### Examples:

### Example 1:

### Nociceptive test procedures

Rats were anaesthetized and the sciatic nerve was exposed through blunt dissection and gently freed from adhering tissues. Four ligatures (5-0 chromic catgut, applied 1-2 mm apart) were then placed around the nerve (Bennett and Xie, 1988). To obtain the desired degree of constriction, the criterion formulated by Bennett and Xie (1988) was applied.

In sciatic nerve-ligatured rats, mechanical nociceptive thresholds (expressed as g) were determined using the Randall and Selitto (1957) paradigm which consists of applying increasing pressures to the nerve-injured hindpaw of unrestrained rats until paw withdrawal and then a squeak (vocalization) are obtained.

TTX was injected s.c. after 15 min prior administration of naloxone or saline at a dose of 3 µg/kg. The concentration of naloxone used in the experiments was 0.5 mg/kg, control animals received the vehicle (0.09% NaCl) under the very same time and environmental conditions. In all cases, each rat received only one treatment (at only one dose of a given compound), and was used once only.

Data are presented as means ± S.E.M. The non parametric Kruskal and Wallis (1952) one-way analysis of variance (ANOVA) was used and comparisons between groups were performed using the Mann-Whitney test. The areas under the time-course curves (AUC) were calculated using the trapezoidal rule. The t-test was used to compare overall effects (AUCs) between two groups. The significance level was set at P < 0.05.

The results shown in figure 1 and 2 clearly demonstrate that administration of tetrodotoxin administrated in combination with naloxone, a µ-opioid antagonist, has a better analgesic effect than TTX administration alone in sciatic nerve-lesioned rats, indicating clear-cut analgesic effects of this drug combination.

## Claims

1. Combination of pharmaceutically active compounds comprising: at least one Compound A) selected from a Sodium channel blocker, and/or of one of its derivatives, optionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate;
and
at least one Compound B) selected from an opioid antagonist optionally in the form of its racemate, and/or one of its derivatives optionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate.

2. Combination of pharmaceutically active compounds consisting of:
at least one Compound A) selected from a Sodium channel blocker, and/or of one of its derivatives, optionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate;
and
at least one Compound B) selected from an opioid antagonist optionally in the form of its racemate, and/or one of its derivativesoptionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate.

3. Combination according to any of claims 1 or 2, **characterized in that** at least one compound A is a sodium channel blocker specific for a TTX-sensitive Sodium channel.

4. Combination according to any of claims 1 or 2, **characterized in that** at least one compound A is a sodium channel blocker specific for a TTX-resistant Sodium channel.

5. Combination according to any of claims 1 or 2, **characterized in that** at least one compound A is selected from Tetrodotoxin and/or at least one of its analogues or derivatives optionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate;
and/or from Saxitoxin and/or at least one of its analogues or derivatives optionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate.

6. Combination according to Claim 5 **characterized in that** at least one compound A is a sodium channel blocker specific for Compound A is selected from tetrodotoxin, optionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate.

7. Combination according to any of Claims 5 or 6 **characterized in that** Compound A is selected from tetrodotoxin in neutral form or as a salt, especially a physiologically acceptable salt.

8. Combination according to any of Claims 5 to 7 **characterized in that** Compound A is selected from tetrodotoxin, its derivative or its analogue which is isolated from a biological source, preferably from fish, especially puffer fish.

9. Combination according to any of Claims 5 to 7 **characterized in that** Compound A is selected from tetrodotoxin, its derivative or its analogue which is synthesized.

10. Combination according to any of claims 1 to 9 **characterized in that** at least one Compound B is an unspecific opioid antagonist, a specific µ-opioid antagonist, a specific ORL1-antagonist, a specific δ-opioid antagonist, or a specific κ-opioid antagonist, including their derivatives optionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate.

11. Combination according to any of claims 1 to 10 **characterized in that** at least one Compound B is a specific δ-opioid antagonist, including the derivatives optionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate.

12. Combination according to any of claims 1 to 10 **characterized in that** at least one Compound B is a specific µ-opioid antagonist, including their derivatives optionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate.

13. Combination according to claim 12 **characterized in that** at least one Compound B is naloxone, Naltrexone, Etonitazenyl isothiocyanate, Naloxonazine dihydrochloride, Clocinnamox mesylate, b-Funaltrexamine hydrochloride, CTOP or CTAP.

14. Combination according to any of claims 1 to 13 **characterized in that** at least one Compound A is Tetrodotoxin or one of its derivatives or analogues and at least one Compound B is a δ-opioid antagonist.

15. Combination according to any of claims 1 to 13 **characterized in that** at least one Compound A is Tetrodotoxin or one of its derivatives or analogues and at least one Compound B is a µ-opioid antagonist.

16. Combination according to claims 15, **characterized in that** Compound A is Tetrodotoxin and Compound B is Naloxone, Naltrexone, Etonitazenyl isothiocyanate, Naloxonazine dihydrochloride, Clocinnamox mesylate, b-Funaltrexamine hydrochloride, CTOP or CTAP.

17. Pharmaceutical formulation comprising a combination according to any of claims 1 to 16 and optionally at least one auxiliary material and/or additive.

18. Use of a combination according to any of Claims 1 to 16 for the production of a medicament for the treatment of pain, especially cancer pain, moderate to severe pain, and neuropathic pain.

19. Use according to Claims 16, in which the neuropathic pain is peripheral neurogenic pain, peripheral neuropathic pain, allodynia, causalgia, Hyperalgesia, Hyperesthesia, Hyperpathia, neuralgia, neuritis, or neuropathy.

20. Use of a combination according to any of Claims 1 to 15 for the production of a medicament for the prophylaxis and/or treatment of drug abuse and/or drug addiction, medicament abuse and/or medicament addiction, preferably drug abuse and/or drug addiction.

21. Use of a combination according to any of Claims 1 to 16 for the production of a medicament for the treatment of anxiety and schizophrenia or schrizophrenic diseases.
